Europäisches Patentamt

European Patent Office

Office européen des brevets

(11) Veröffentlichungsnummer: **0 053 647**
**B1**

(12) **EUROPÄISCHE PATENTSCHRIFT**

(45) Veröffentlichungstag der Patentschrift:
**07.01.87**

(21) Anmeldenummer: **81101935.5**

(22) Anmeldetag: **16.03.81**

(51) Int. Cl.⁴: **A 61 N 1/10, A 61 N 1/40**

(54) **Impulsfelderzeugendes Kompaktgerät.**

(30) Priorität: **06.12.80 DE 8032590 U**

(43) Veröffentlichungstag der Anmeldung:
**16.06.82 Patentblatt 82/24**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

(56) Entgegenhaltungen:
**DE - A - 1 753 256**
**DE - A - 1 920 137**
**DE - A - 2 319 357**
**DE - B - 1 217 576**
**US - A - 3 531 150**

(73) Patentinhaber: **Baumgartner, Reinhard,**
**Dipl.-Wirtsch.-Ing., Dipl.-Kaufm., Henschelstrasse 1,**
**D-8000 München 60 (DE)**

(72) Erfinder: **Zöbisch, Edmund, Augustenstrasse 24,**
**D-8000 München 2 (DE)**
Erfinder: **Zöbisch, Klaus, Hochwaldstrasse 28,**
**D-8011 Baldham (DE)**
Erfinder: **Baumgartner, Reinhard Dipl.-Wirt-Ing.,**
**Herschelstrasse 1, D-8000 München 60 (DE)**

(74) Vertreter: **Schieschke, Klaus, Dipl.-Ing. et al,**
**Patentanwälte Dipl.-Ing. E. Eder Dipl.-Ing. K. Schieschke**
**Elisabethstrasse 34, D-8000 München 40 (DE)**

## Beschreibung

Die Erfindung betrifft ein Gerät zur Erzeugung eines bioklimatisch wirksamen elektrischen Feldes mit einem einen Generator und eine Elektrode aufnehmenden Gehäuse.

Es ist bekannt, dass das Wohlbefinden des Menschen durch das elektrische Feld des Ortes, an dem er sich befindet, beeinflusst wird. Während der Mensch in der freien Natur dem natürlichen elektrischen Feld ausgesetzt ist, bewirkt die elektrische Abschirmung in geschlossenen Räumen, wie in Zimmern und Kraftfahrzeugen, dass das elektrische Feld fehlt oder nur abgeschwächt auf den Menschen einwirkt. Um in derartigen Räumen ein dem natürlichen entsprechendes bioelektrisches Klima herzustellen, werden Geräte verwendet, die ein mit Impulsen überlagertes Gleichfeld erzeugen. Die positive Wirkung dieses künstlichen elektrischen Feldes auf den Menschen ist nachweisbar; es wird insoweit auf die DE-PS 25 07 783 verwiesen.

Aus der DE-OS 19 20 137 ist ein derartiges Gerät bekannt. Dieses Gerät ist jedoch nicht transportabel, sein Generator nicht zur Erzeugung von für das Wohlbefinden und die Leistungsfähigkeit des Menschen bedeutsame Impulsspannung ausgelegt, die Elektrode ist aufwendig ausgestaltet.

Der Erfindung liegt die Aufgabe zugrunde, ein Gerät zur Erzeugung eines bioklimatisch wirksamen elektrischen Feldes zu schaffen, welches bei einfachem Aufbau das für das Wohlbefinden und die Leistungsfähigkeit des Menschen erforderliche elektrische Feld aufbaut und mit einer einfach konstruierten Elektrode versehen ist.

Erfindungsgemäss wird diese Aufgabe dadurch gelöst, dass das aus Kunststoff bestehende Gehäuse eine Batterie aufnimmt, dass der Generator neben der Gleichspannung zur Erzeugung von Impulsspannung ausgelegt ist, und dass die Elektrode aus einer leitfähigen Beschichtung der Innenfläche eines Gehäusedeckels besteht.

Weitere Merkmale und Vorteile der Erfindung ergeben sich aus der nachfolgenden Beschreibung, in der ein Ausführungsbeispiel anhand der Zeichnung im einzelnen erläutert ist. Dabei zeigt:

Fig. 1 eine Ansicht eines Ausführungsbeispiels der Erfindung, etwa massstäblich;

Fig. 2 einen Schnitt entlang der Linie A–A von Fig. 1, etwa massstäblich;

Fig. 3 die Anbringung des Gerätes an der Sonnenblende eines Kraftfahrzeuges;

Fig. 4 einen Schnitt entlang der Linie B–B von Fig. 3;

Fig. 5 die Anbringung des Gerätes auf einem Tischständer;

Fig. 6 einen Schnitt entlang der Linie C–C von Fig. 5, etwa in der Grösse 1:4.

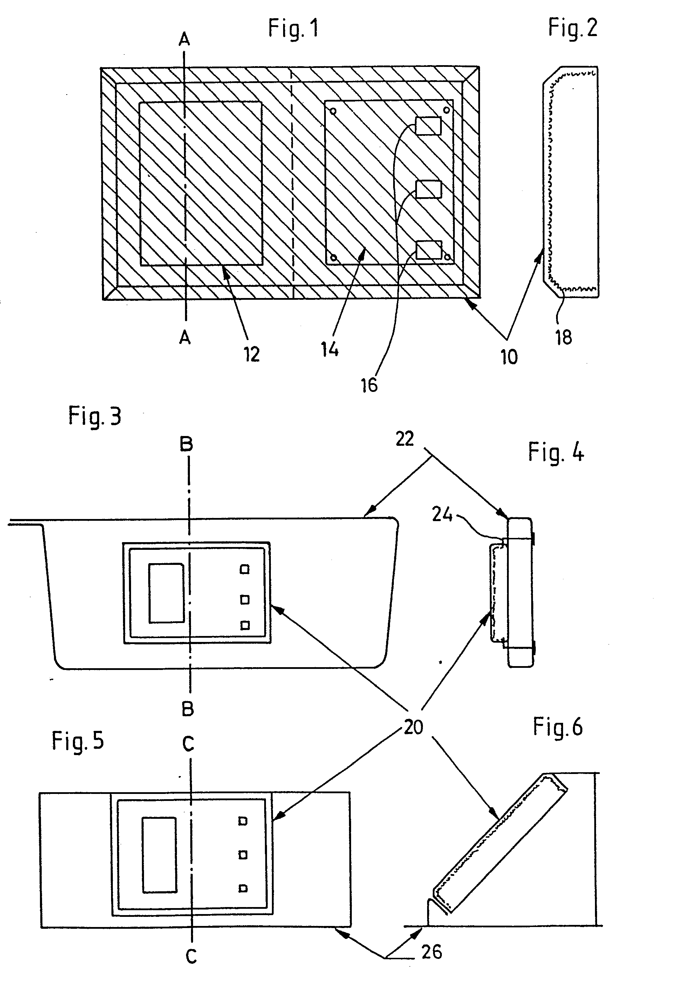

Wie Fig. 1 erkennen lässt, befinden sich in einem Gehäuse 10, das aus Kunststoff hergestellt ist, eine Batterie 12, sowie der die elektrische Gleichspannung und die diese überlagernden Impuls erzeugende Generator 14 mit den Gehäusedeckel durchdringenden Bedienungselementen 16.

In Fig. 2 ist eine Elektrode 18 in Form der Innenbeschichtung des Gehäusedeckels mit einem leitfähigen Material zu erkennen.

In Fig. 3 und 4 ist die Befestigung des Gerätes 20 auf einer Sonnenblende 22 eines Kraftfahrzeuges mittels einer Klemmvorrichtung 24 zu erkennen. In vielen Anwendungsfällen kann es aber auch besonders vorteilhaft sein, das erfindungsgemässe Gerät zwischen den Sonnenblenden eines Kraftfahrzeuges oder dergl. mittels einer Selbstklebefolie oder dergl. zu befestigen.

Fig. 5 und 6 stellen die Anbringung des Gerätes auf einem Tischständer 24 dar.

Die Vorteile der Erfindung gegenüber den bekannten immobilen Impulsfeldgeräten liegen in den weit geringeren Produktionskosten, der Möglichkeit, das etwa zigarrenschachtel-grosse Gerät ständig bei sich zu haben und der Einwirkung des Gerätes nur auf eine Person, die die Feldparameter individuell für sich optimieren kann.

## Patentanspruch

Gerät zur Erzeugung eines bioklimatisch wirksamen elektrischen Feldes, mit einem einen Generator und eine Elektrode aufnehmenden Gehäuse, dadurch gekennzeichnet,

dass das aus Kunststoff bestehende Gehäuse (10) eine Batterie (12) aufnimmt,

dass der Generator (14) neben der Erzeugung einer Gleichspannung zur Erzeugung einer Impulsspannung ausgelegt ist, und

dass die Elektrode (18) aus einer leitfähigen Beschichtung der Innenfläche eines Gehäusedeckels besteht.

## Claim

An appliance for generating a bioclimatically effective electrical field comprising a housing, which accommodates a generator, and an electrode, characterised in that the housing (10) consists of plastics material and accommodates a battery (12), in that the generator (14) is designed for generating an impulse voltage as well as for generating a direct voltage, and in that the electrode (18) consists of a conductive coating of an inner surface of a housing cover.

## Revendication

Dispositif générateur de champs impulsionnels comportant un boîtier contenant un générateur et une électrode, caractérisé en ce que le boîtier (10) en matière plastique renferme une batterie (12), en ce que le générateur (14) est conçu pour produire, outre une tension continue, une tension impulsionnelle et en ce que l'électrode (18) est constituée par un revêtement conducteur de la face intérieure d'un couvercle du boîtier.

Fig. 1

Fig. 2

A — A

12  14  16  10  18

Fig. 3

Fig. 4

22  24  20

Fig. 5

Fig. 6

B — B

C — C

20  26